# EUROPEAN PATENT APPLICATION

(11) **EP 0 534 380 A1**
(43) Date of publication of application: **31.03.1993**
(21) Application number: 92116235.0
(22) Date of filing: 23.09.1992
(51) Int. Cl.: G01N 33/58, G01N 33/533, G01N 21/76

(54) **Agent and method for enhancing chemiluminescence**

(30) Priority: 24.09.1991 JP 243253/91
(71) Applicant: KYOTO DAIICHI KAGAKU CO., LTD., Kyoto-shi Kyoto-fu (JP)
(72) Inventor: Takama, Toshio, c/o Kyoto Daiichi Kagaku Co., Ltd., Kyoto-shi, Kyoto-fu (JP)
(74) Representative: von Kreisler, Alek, Dipl.-Chem.

(57) **Abstract**

A quaternary phosphate salt of the formula: 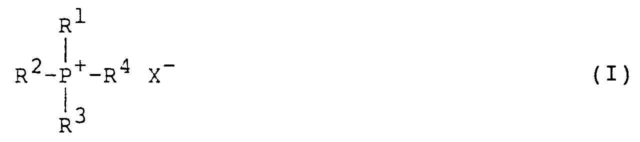
wherein one of R¹, R², R³ and R⁴ is an alkyl or alkenyl group having 8 to 20 carbon atoms, the rest groups are the same or different and each an alkyl or alkenyl group having 1 to 8 carbon atoms, and X⁻ is a halogen ion is useful as an agent for enhancing chemiluminescence which is generated through oxidation of an acridinium ester in various assays.

## Description

### BACKGROUND OF THE INVENTION

### Field of the Invention

The present invention relates to an agent and method for enhancing chemiluminescence. More particularly, the present invention relates to an agent and method for enhancing chemiluminescence which is generated through oxidation of an acridinium ester.

### Description of the Related Art

Chemiluminescence generated through oxidation of an acridinium ester is used for qualitatively or quantitatively assay an object ingredient in various immunoassays. That is, the object ingredient or a compound which bonds with the object ingredient is labeled with the acridinium ester and subjected to the immunoassay. An oxidizing agent such as hydrogen peroxide is added to the assay system to cause chemiluminescence. Then, an intensity of chemiluminescence is measured to determine the presence and a concentration of the object ingredient.

To enhance the chemiluminescence generated through the oxidation of the acridinium ester, it has been proposed to add a nonionic surfactant, a sulfate salt with a primary alcohol or a quaternary ammonium salt to a chemiluminescence system (see U.S. Patent No. 4,927,769).

However, the proposed enhancers such as the nonionic surfactant, the sulfate salt with the primary alcohol or the quaternary ammonium salt have still insufficient chemiluminescence enhancing effects, and a chemiluminescence enhancer having a higher sensitizing rate has been desired.

### SUMMARY OF THE INVENTION

One object of the present invention is to provide an agent for enhancing chemiluminescence generated through the oxidation of the acridinium ester in the immunoassay.

Another object of the present invention is to provide a method for enhancing chemiluminescence generated through the oxidation of the acridinium ester in the immunoassay.

According to the present invention, there are provided an agent for enhancing chemiluminescence comprising a quaternary phosphate salt of the formula: 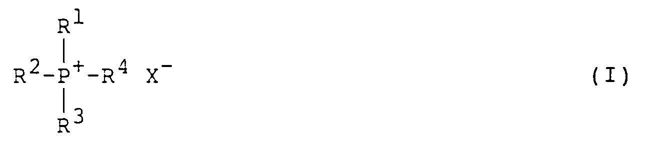
wherein one of R¹, R², R³ and R⁴ is an alkyl or alkenyl group having 8 to 20 carbon atoms, the rest groups are the same or different and each an alkyl or alkenyl group having 1 to 8 carbon atoms, and X⁻ is a halogen ion, and a method for enhancing chemiluminescence using said agent.

### DETAILED DESCRIPTION OF THE INVENTION

Among the alkyl or alkenyl groups having 8 to 20 carbon atoms in the formula (I), an alkyl group having 14 to 18, in particular 16 carbon atoms is preferred. Among the alkyl or alkenyl groups having 1 to 8 carbon atoms, an alkyl group having 4 to 8, in particular 4 carbon atoms is preferred.

Preferred examples of the quaternary phosphonium salt (I) are cetyltri-n-butylphosphonium halide, in particular cetyltri-n-butylphosphonium bromide, eicosanetributylphosphonium bromide, tridecanetributylphosphonium bromide, hexadecanetributylphosphonium bromide, hexadecanetrihexylphosphonium bromide and the like.

The chemiluminescence assay using oxidation of the acridinium ester may be carried out in the same manner as in the conventional immunoassay except that the above quaternary phosphate salt is added to the system. The conventional immunoassay is disclosed, for example, in U.S. Patent No. 4,927,769, the disclosure of which is hereby incorporated by reference.

One of typical assays using the acridinium ester is as follow: an acridinium ester-labeled antibody and a biotin-labeled antibody which identify different epitopes of the same antigen are reacted with an antigen in one step to form an antigen-antibody complex. A mixture containing the complex is added to a glass filter to which avidin is bonded and kept standing. After B/F separation, an acidic buffer is added to the glass filter and kept standing, and an alkaline solution is added thereto. Then, an amount of chemiluminescence is measured. The quaternary phosphate salt is added to the acidic buffer or the alkaline solution.

Typical examples of the acridinium eater are disclosed in U.S. Patent No. 4,927,769, in particular, columns 3 and 4, the disclosure of which is hereby incorporated by reference.

A concentration of the quaternary phosphate salt (I) is usually at least 0.01 % by weight, preferably at least 0.5 % based on the weight of a reaction mixture for chemiluminescence reaction. When the quaternary phosphate salt (I) is added to an agent which is lastly added to the reaction mixture (e.g. an alkaline liquid) among reagents to be used for the chemiluminescence reaction, a higher enhancing effect is achieved.

By the addition of the quaternary phosphate salt (I) to the chemiluminescence system of the acridinium ester, the intensity of chemiluminescence is increased. In addition, if a concentration of the objective ingredient in a sample is low, an accurate assay can be performed due to the enhancing effect of the quaternary phosphate salt (I).

### PREFERRED EMBODIMENTS OF THE INVENTION

The present invention will be illustrated by the following Examples, which will not limit the scope of the present invention.

### Example 1 and Comparative Examples 1 and 2

In 0.1 M phosphate buffer, Acridinium I (manufactured by Dojin Chemical, Co., Ltd.) was reacted and bonded with anti-β₂-microglobulin antibody (BM-012 manufactured by Boehlinger Manheim) in a molar ratio of 5:1 (pH = 8.0) to obtain an Acridinium I-labeled antibody.

Separately, in 0.1 M aqueous NaHCO₃, N-hydroxysuccinimde-biotin (manufactured by Pias) was reacted with anti-β₂-microglobulin antibody (BM-011 manufactured by Boehlinger Manheim) in a molar ratio of 27:1 to label the antibody with biotin.

Two labeled antibodies and β₂-microglobulin (manufactured by Cosmobio) were mixed to proceed an antigen-antibody reaction for 10 minutes. To trap a generated antigen-antibody complex, the reaction mixture (50 µl) was added to an avidin-bonded glass filter having a diameter of 9 mm and kept standing for 5 minutes. Thereby, the complex was trapped by the glass filter through avidin-biotin bondings. Then, the glass filter was washed with a physiological saline buffered with a phosphate under suction by a Buchner funnel to carry out B/F separation.

After the B/F separation, to the glass filter, 15 µl of 0.01 M citric acid-phosphate buffer (pH 4.0) containing 3 % of hydrogen peroxide and kept standing for 10 minutes. Then, 0.05 N aqueous NaOH (25 µl) was added to the glass filter and an intensity of chemiluminescence was measured by a system for detecting weak light of chemiluminescence (manufactured by Kyoto Daiichi Kagaku).

In the above assay system which is referred to as a "CLIA" system, the following chemiluminescence enhancer was added to the 0.01 M citric acid-phosphate buffer containing 3 % of hydrogen peroxide or 0.05 N aqueous NaOH, and the enhancing effects were compared.

The results are as follows:

### Comparison of enhancing effects in CLIA system (I)

(The enhancer being added to the 0.01 M citric acid-phosphate buffer containing 3 % of hydrogen peroxide)

### Example 1

| Cetyltri-n-butylphosphonium bromide | Intensity (counts) | Enhancing ratio (times) |
|---|---|---|
| 0 % (no addition) | 28,431 | (1.00) |
| 0.1 % | 57,536 | 2.02 |
| 1 % | 66,103 | 2.32 |
| 5 % | 67,367 | 2.36 |

### Comparative Example 1

| Cetyltrimethylammonium chloride | Intensity (counts) | Enhancing ratio (times) |
|---|---|---|
| 0 % (no addition) | 33,423 | (1.00) |
| 0.1 % | 39,738 | 1.18 |
| 1 % | 52,750 | 1.57 |
| 5 % | 57,843 | 1.73 |

### Comparative Example 2

| Tween 20 | Intensity (counts) | Enhancing ratio (times) |
|---|---|---|
| 0 % (no addition) | 44,142 | (1.00) |
| 0.1 % | 42,838 | 0.97 |
| 1 % | 49,116 | 1.11 |
| 5 % | 67,402 | 1.52 |

### Comparison of enhancing effects in CLIA system (II)

(The enhancer being added to the 0.05 N aqueous NaOH)

### Example 1

| Cetyltri-n-butylphosphonium bromide | Intensity (counts) | Enhancing ratio (times) |
|---|---|---|
| 0 % (no addition) | 29,592 | (1.0) |
| 0.1 % | 153,570 | 5.1 |
| 1 % | 196,950 | 6.6 |
| 5 % | 201,322 | 6.8 |

### Comparative Example 1

| Cetyltrimethylammonium chloride | Intensity (counts) | Enhancing ratio (times) |
|---|---|---|
| 0 % (no addition) | 25,232 | (1.0) |
| 0.1 % | 145,774 | 5.7 |
| 1 % | 145,929 | 5.7 |
| 5 % | 172,420 | 6.8 |

### Comparative Example 2

| Tween 20 | Intensity (counts) | Enhancing ratio (times) |
|---|---|---|
| 0 % (no addition) | 29,592 | (1.00) |
| 0.1 % | 31,296 | 1.05 |
| 1 % | 54,704 | 1.84 |
| 5 % | 66,457 | 2.2 |

### Example 2

### Measurement of intensities of chemiluminescence in a solution system

In 0.1 M phosphate buffer (pH = 8.0), Acridinium I (manufactured by Dojin Chemical Co., Ltd.) was reacted with a monoclonal antibody MAK 33 (manufactured by Boehlinger Manheim) in a molar ratio of 5:1 to obtain an Acridinium I-labeled antibody. The Acridinium I labeled-antibody (10 µg/ml) was diluted by 500 times with 0.1 M citric acid-phosphate buffer (pH = 5.0). The diluted liquid (400 µl) was poured a cuvette for luminescence measurement. To the cuvette, 1 N aqueous NaOH containing 0.3 % of hydrogen peroxide (100 µl) was added and an intensity of chemiluminescence was measured by a luminescent detector TD-4000 (manufactured by Labo-Science).

The following chemiluminescence enhancer was added to the 0.1 M citric acid-phosphate buffer or 1 N aqueous NaOH containing 0.3 % of hydrogen peroxide, and the enhancing effects were compared.

### Comparison of enhancing effects in a solution system (1)

(The enhancer being added to the 0.01 M citric acid-phosphate buffer in a concentration of 0.5 % by weight)

| Enhancer | Intensity (rlu) | Enhancing ratio (times) |
|---|---|---|
| (No addition) | 150 | (1.0) |
| Cetyltri-n-butylphosphonium bromide | 303 | 2.0 |
| Tween 20 | 456 | 3.0 |
| Cetyltrimethylammonium chloride | 303 | 2.0 |
| Sodium laurylbenzenesulfonate | 954 | 6.3 |

### Comparison of enhancing effects in a solution system (2)

(The enhancer being added to the 1 N aqueous NaOH containing 0.3 % of hydrogen peroxide in a concentration of 0.5 % or 0.2 % by weight)

| Enhancer | Intensity (rlu) | | | | | |
|---|---|---|---|---|---|---|
| (concentration) | 0.5 % | 0.2 % | 0.5 % | 0.2 % | 0.5 % | 0.2 % |
| (No addition) | 270 | 270 | 200 | 200 | 198 | 198 |
| Cetyltri-n-butylphosphonium bromide | 624 (2.3) | 634 (2.3) | | | | |
| Tween 20 | | | 240 (1.2) | 234 (1.17) | | |
| Cetyltrimethylammonium chloride | | | | | 461 (2.3) | 453 (2.2) |
| Sodium laurylbensenesulfonate | [insoluble] | | [insoluble] | | [insoluble] | |
| Note: The values in the parentheses were the enhancing ratios (times). | | | | | | |

## Claims

1. An agent for enhancing chemiluminescence comprising a quaternary phosphate salt of the formula: 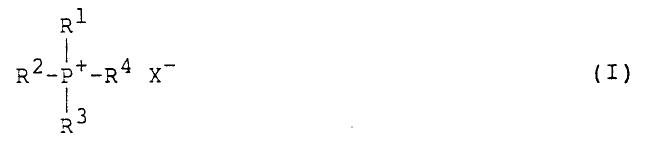 wherein one of R¹, R², R³ and R⁴ is an alkyl or alkenyl group having 8 to 20 carbon atoms, the rest groups are the same or different and each an alkyl or alkenyl group having 1 to 8 carbon atoms, and X⁻ is a halogen ion.

2. The agent for enhancing chemiluminescence according to claim 1, wherein the alkyl or alkenyl group having 8 to 20 carbon atoms in the formula (I) is an alkyl group having 14 to 18, in particular 16 carbon atoms.

3. The agent for enhancing chemiluminescence according to claim 1, wherein the alkyl or alkenyl group having 1 to 8 carbon atoms is an alkyl group having 4 to 8, in particular 4 carbon atoms.

4. The agent for enhancing chemiluminescence according to claim 1, wherein the quaternary phosphate salt (I) is cetyltri-n-butylphosphonium halide.

5. The agent for enhancing chemiluminescence according to claim 4, wherein cetyltri-n-butylphosphonium halide is cetyltri-n-butylphosphonium bromide.

6. A method for enhancing chemiluminescence comprising adding a quaternary phosphate salt of the formula: 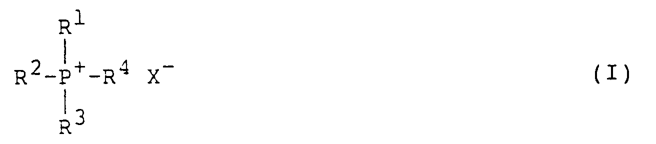 wherein one of R¹, R², R³ and R⁴ is an alkyl or alkenyl group having 8 to 20 carbon atoms, the rest groups are the same or different and each an alkyl or alkenyl group having 1 to 8 carbon atoms, and X⁻ is a halogen ion to an assay system which utilizes the chemiluminescence.

7. The method according to claim 6, wherein the chemiluminescence is generated through oxidation of an acridinium ester.

8. The method for enhancing chemiluminescence according to claim 6, wherein the alkyl or alkenyl group having 8 to 20 carbon atoms in the formula (I) is an alkyl group having 14 to 18, in particular 16 carbon atoms.

9. The method for enhancing chemiluminescence according to claim 6, wherein the alkyl or alkenyl group having 1 to 8 carbon atoms is an alkyl group having 4 to 8, in particular 4 carbon atoms.

10. The method for enhancing chemiluminescence according to claim 6, wherein the quaternary phosphate salt (I) is cetyltri-n-butylphosphonium halide.

11. The method for enhancing chemiluminescence according to claim 10, wherein cetyltri-n-butylphosphonium halide is cetyltri-n-butylphosphonium bromide.

12. Use of a quaternary phosphate salt of the formula: 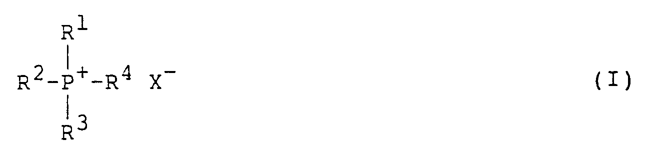 wherein one of R¹, R², R³ and R⁴ is an alkyl or alkenyl group having 8 to 20 carbon atoms, the rest groups are the same or different and each an alkyl or alkenyl group having 1 to 8 carbon atoms, and X⁻ is a halogen ion, as an agent for enhancing chemiluminescence.

13. The use according to claim 10, wherein the chemiluminescence is generated through oxidation of an acridinium ester.
